(19) Europäisches Patentamt
European Patent Office
Office européen des brevets

(11) Publication number: 0 101 285 B1

# (12) EUROPEAN PATENT SPECIFICATION

(45) Date of publication of patent specification: 31.07.91 (51) Int. Cl.$^5$: C12N 5/00

(21) Application number: 83304573.5

(22) Date of filing: 08.08.83

## (54) A method for culturing and isolating cells.

(30) Priority: 09.08.82 JP 138153/82

(43) Date of publication of application:
22.02.84 Bulletin 84/08

(45) Publication of the grant of the patent:
31.07.91 Bulletin 91/31

(84) Designated Contracting States:
DE FR GB IT

(56) References cited:
WO-A-80/01350
CH-A- 423 095
FR-A- 2 396 083

CHEMICAL ABSTRACTS, vol. 99, no. 3, 18th July 1983, page 433, abstract no. 20299b, Columbus, Ohio, US; S. KASAI et al.: "Enhanced effect of chemically modified collagen substratum on attachment and growth of fibroblasts in serum-free medium"

CHEMICAL ABSTRACTS, vol. 77, no. 6, 6th November 1972, page 149, abstract no. 123496p, Columbus, Ohio, US; G.M. CLEATOR et al.: " Probable role of collagen when used as a growth surface for cell culture"

Trans.Amer.Soc.Artif.Int.Organs, vol. 22, pp. 261-267(1976)

J.Cell.Sci.,vol. 38, pp. 267-281(1979)

Develop.Biol.Stanard, vol. 41, pp. 109-116(1980)

(73) Proprietor: KOKEN CO. LTD.
5-18 Shimoochiai 3-chome
Shinjuku-ku Tokyo(JP)

(72) Inventor: Kasai, Shunji
5-6-203, Fujisaka Nishi-machi
Hirakata-shi Osaka(JP)
Inventor: Akaike, Toshihiro
4-15-23, Shimohouya
Houya-shi Tokyo(JP)
Inventor: Miyata, Teruo
3-6-29-314, Shimoochiai
Shinjuku-ku Tokyo(JP)

(74) Representative: Crampton, Keith John Allen et al
D. YOUNG & CO. 10 Staple Inn
London WC1V 7RD(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to the use of collagen-derived substrates for cell culture.

Collagen exists in various organs such as blood vessels, skin, liver, pancreas and kidney as the main ccmponent of the connective tissue. It functions as a supporting substance for cell growth and plays an important role as the matrix for manifestation of the functions of tissues and organs. Atelocollagen, which is prepared by pepsin treatment of acid-soluble collagen or insoluble collagen, has been widely used as a substrate for animal cell culture. Different mechanisms in the attachment of cells tò native and denatured collagen are described by Schor and Court, J. Cell Sci., 38, 267-281 (1979).

Recently, studies on artificial organs have been developed. In particular, studies on artificial organs incorporating cells peculiar to each organ, that is to say, studies on hybrid organs, have been actively conducted. For instance, the development of a hybrid organ for the liver, in which hepatocytes are incorportated for maintaining cell metabolic activity, has been attempted. In this case, it is important to stick the cells to the substrate while retaining the cell activity exhibited in vivo, and the type of substratum used is a fundamental consideration. As a substratum for liver cells, collagen extracted from the liver is reported to be best. (M. Rjkind et al, Connective Tissue Biomatrix: Its Isolation for Longterm Culture of Normal Rat Hepatocytes, J. Cell Biol. 87, 255 (1980)).

Miyata et al (Trans. Amer. Soc. Artif. Int. Organs, vol. XXII, pages 261-267, 1976) describes the deposition of blood platelets and fibrin on chemically modified collagen hollow fibres in comparison with unmodified collagen fibres. For their studies, Miyata et al used methylated enzyme-solubilized collagen and succinylated enzyme-solubilized collagen, and they found that blood platelets and fibrin were deposited more rapidly and in greater numbers on methylated surfaces and more slowly and to a lesser extent on succinylated surfaces compared to unmodified surfaces.

Hirtenstein et al (Develop. Biol. Standard, vol. 46, pages 109-116, 1980) describes various types of microcarrier beads for animal cell culture and they indicate that cells can be cultured on surfaces which possess either negative or positive charges. There is no indication in this article that chemically modified collagen could be used for cell culture.

So-called methods of cell technology such as cell culture and cell isolation currently occupy one of the important scientific technical fields in the advancement of life science. That is to say, effective cell culture and the techniques for isolating cells are the most important techniques in cell technology for the production of biologically active cell-derived substances. In the culture of animal cells, it is important to select a substratum with good adhesion for the cells in order to keep the original cell activity or in order to make the cells proliferate, and it is usually necessary to add bovine foetal serum to the culture medium in order to maintain the cell activity or cause cells to proliferate. However, recently, owing to difficulties in and expense of obtaining bovine foetal serum, the establishment of a serum-free cell culture method has been sought. Recently, fibronectin, which is a kind of protein, has been noticed as a cell attachment factor and cell culture has been established in a fibronectin system instead of bovine foetal serum.

According to the present invention, there is provided a method for culturing and isolating cells comprising causing animal cells to adhere to succinylated collagen wherein more than 20 mol % of the total amino groups of collagen have been succinylated, or to esterified collagen wherein more than 20 mol % of the total carboxyl groups of collagen have been esterified, in a culture medium, incubating the animal cells and subsequently treating the animal cells with a bivalent cationic chelating agent to isolate and recover the cells.

The use of succinylated or esterified collagen in accordance with the invention enhances attachment of animal cells. For instance, mice fibroblasts (L-cells) or macrophages (Mø) adhere to the modified collagen much better than to unmodified collagen in the presence or absence of bovine foetal serum and the modified collagen is thus superior as the substratum for cell culture. The use of succinylated or esterified collagen as a substratum is convenient in a serum-free culture method, so that cell culture can be effected at a cheaper cost and the difficulties involved in purchasing bovine foetal serum can be avoided. When fibronectin is used in place of bovine foetal serum, the attachment and proliferation of cells resulting from use of the chemically modified collagen substratum in accordance with the present invention are far superior to those arising from the use of an unmodified collagen substratum. Furthermore, adhering cells may be detached from the modified collagen with a high efficiency so that the isolation and recovery of macrophages, which have been desired in the fields of immunology and tumorology, can be selectively made without cell injury.

All the figures of the accompany drawings are graphs.

Fig. 1 shows the relations between the incubation time and cell adhesion rate of mice fibroblast (L-cells) by using various collagen substrata in the presence of bovine foetal serum.

Fig. 2 shows similar relations to those of Fig. 1 in the absence of bovine foetal serum.

Fig. 3 shows the relation between the incubation time and the number of cells of mice fibroblast (L-cells).

Fig. 4 shows the relation between the incubation time and the cell adhesion rate of macrophages by using various collagen substrates in the presence of bovine foetal serum.

Fig. 5 shows similar relations to Fig. 4 in the absence of bovine foetal serum.

Native collagen, e.g. unmodified collagen, bears positive and negative charges in the side groups of polypeptide chains. Basic amino acids, e.g. arginine, lysine and histidine provide positive charges and acidic amino acids, e.g. glutamic and aspartic acids, provide negative charges. The numbers of basic and acidic amino acid residues of unmodified collagen are 85 and 78 respectively per 1000 residues of amino acids, therefore basic amino acid is in an excess of 7.

In one aspect, the invention employs succinylated collagen. As shown in the following scheme (1), a succinylation reaction is carried out by introducing a succinyl group on the ε-amino group of collagen with succinic anhydride and thus converting a free amino group to a free carboxyl group.

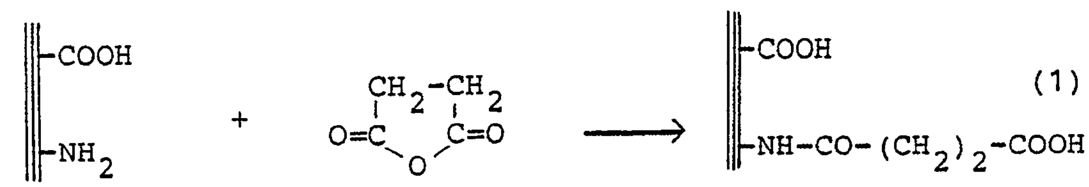

(1)

Collagen Succinic anhydride Succinylated collagen

By this reaction, the succinylated collagen is rich in negative charge. When succinylation has proceeded to completion, the resulting succinylated collagen has negative charges of 49 per 1,000 residues in excess at pH7.

More than 20% and particularly more than 40% of all the amino groups of collagen are succinylated. If the degree of succinylation is inadequate, the adhering property of cells cannot be improved sufficiently.

In another aspect, the invention employs esterified collagen. Esterification takes place by reaction of the carboxyl groups with an alcohol in the presence of an acid catalyst. This reaction is represented by the following reaction scheme (2),

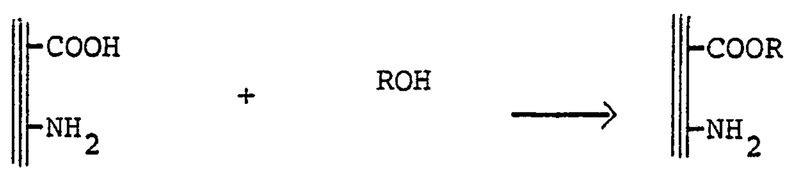

(2)

Collagen Alcohol Esterified collagen

In scheme (2), R represents a straight or branched monovalent aliphatic hydrocarbon residue, preferably a methyl or ethyl group. When esterification is complete, negative charges are eliminated, and therefore, the positive charges of esterified collagen are in excess by 85 per 1,000 residues at pH7. More than 20%, paticularly more than 40%, of all carboxyl groups of collagen are esterified. If the degree of esterification is inadequate, the effect of enhancing cell attachment cannot be obtained.

Another method of obtaining the chemically modified collagen rich in positive charges involves esterifying the carboxyl groups by coupling them with nucleophilic groups via water-soluble carbodiimide, and, as a method of modifying the positive charge on the side chain of arginine, reaction with, for example butandione, 2,4-cyclohexadione or phenylglyoxal may be used.

The collagen material to be modified is preferably atelocollagen, which is prepared by solubilizing the insoluble collagen of bovine corium with pepsin, but acid-soluble collagen, which can be extracted with only acid, and insoluble collagen can also be used.

Animal cells that are incubated after adhering to the chemically modified collagen substratum in accordance with the present invention can be isolated and recovered. For instance, macrophages can be

isolated from a mixed system of lymphocytes and macrophages, because only macrophages adhere selectively to the chemically modified collagen substratum when incubated in the presence of bovine foetal serum or fibronectin. Selectively adhering macrophages can be released and recovered in high yield by treatment with a bivalent cationic chelating agent such as EDTA (ethylenediaminetetraacetic acid) or EGTA (ethylene glycol bis (β-aminoethyl ether) N,N,N',N'-tetraacetic acid) without cell injury. By this method macrophages are highly selectively isolated and recovered from the above mixed system. When unmodified collagen is used as a substratum it is impossible to isolate and recover macrophages because they do not efficiently adhere to unmodified collagen. In the absence of bovine foetal serum or fibronectin, it is difficult to detach macrophages even when they are treated with a bivalent cationic chelating agent. Then, the highly selective isolation of macrophages can be attained by using the chemically modified collagen substratum rich in negative or positive charge, preferably in the presence of bovine foetal serum or fibronectin, and by treating the adhered macrophages with a bivalent cationic chelating agent.

The following Examples illustrate the present invention. All parts and percentages in the Examples are on a weight basis unless otherwise indicated. The word "Millipore" is a trade mark.

## Example 1

Calf skin corium was crushed and then washed in 5% aqueous sodium chloride solution, followed by 1% aqueous sodium bicarbonate solution, to remove soluble materials, and then water. Thereafter pepsin was added at a ratio of 0.5% of dried collagen to skin corium suspension (pH 3.0) acidified with hydrochloric acid and insoluble collagen was dissolved at a temperature between 20 °C and 25 °C with stirring. After dissolution, the solution was filtered sucessively through filter paper and Millipore filter of 1 μm and 0.45 μm aperture respectively and was allowed to stand overnight after adjusting the pH to 11.0 to inactivate pepsin. Then the pH of the solution was adjusted to 7.0-7.5, and the resulting precipitates of atelocollagen were collected by centrifuging and washed in water. These precipitates were redissolved in dilute hydrochloric acid of pH 3, reprecipitated at pH 7.0-7.5, and collected by centrifuging.

10 g (dry weight) of atelocollagen was dispersed in 2 litres of a borax buffer solution of pH 10.0 and was succinylated by slowly adding a 5% solution in acetone of succinic anhydride. During the reaction, 1 N sodium hydroxide solution was dripped in to keep the pH 10. As the reaction preceeded, the atelocollagen precipitate dispersed in the solution was dissolved. After the addition of anhydrous succinic acid was completed, the pH of the solution was adjusted to 4.5 to precipitate succinylated collagen. The precipitate was collected by centrifuge, washed in water, redissolved in dilute hydrochloric acid of pH 3, reprecipitated at pH 4.5, and washed in water to produce purified succinylated atelocollagen, which had a succinylation value of 80-90%.

10 g of atelocollagen that had been dried over silica gel under vacuum was immersed in anhydrous methanol containing 0.1N hydrochloric acid for 1 week. Methylated atelocollagen was formed. Its methyl esterification value was 60-80%.

Succinylated atelocollagen, methylated atelocollagen and non-modified atelocollagen were dissolved in dilute hydrochloric acid of pH 3 to make 0.3% solutions and the respective solutions were filtered through a 0.45 μm Millipore filter to remove bacteria. Plastics petri dishes were coated with each of the collagen solutions from which bacteria had been removed, and after drying aseptically by air, the dishes were irradiated by ultra-violet rays for 1 hour to crosslink the collagen. Furthermore, the collagen coated petri dishes were washed with Hank's salt solution three times.

$2.5 \times 10^4$ cells of mice fibroblast (L-cells) were seeded per dish for culture and were incubated in culture media. Eagle culture medium containing 10% bovine foetal serum and Eagle culture medium not containing that serum were used as culture media. The incubation was carried out at 37 °C under 5% $CO_2$ gaseous phase. After incubating for a certain time, the culture liquid was discarded, the cells were washed in the same culture medium three times, and the cells detached from collagen substratum were washed out. The numbers of cells adhering to the substratum were calculated by counting the numbers of the cells washed out.

The relations of incubation time with the adhesion rate of cells were shown in Fig. 1 and Fig. 2. Fig. 1 shows the result in the presence of bovine foetal serum and Fig. 2 the result in its absence. In Figs. 1 and 2, the marks Δ shows the result obtained from unmodified atelocollagen, the mark 0 shows the result obtained from succinylated atelocollagen the mark X shows the result obtained from methylated atelocollagen.

As is clear from Figs. 1 and 2, methyl-esterified atelocollagen and succinylated atelocollagen adhered to L-cells at a higher rate than unmodified atelocollagen (control) in the presence and absence of bovine foetal serum.

Example 2

A proliferation test of L-cells was conducted in the presence and absence of fibronectin by using culture dishes coated respectively with succinylated atelocollagen, methylated atelocollagen, and unmodified atelocollagen, which had been prepared by the method described in Example 1. To the culture dishes coated with collagens, 0.5 ml of Hank's salt solution containing 100 $\mu$g/ml of human plasma fibronectin was added, and after allowing it to stand at 4 °C overnight and washing with Hank's salt solution twice, fibronectin was fixed to collagen substratum.

1.5 x $10^4$ cells of L-cells per culture dish were incubated by using serum-free Eagle culture medium, and the relation of the incubation time to the numbers of the cells was tested. The relation of the incubation time with the numbers of the cells was similarly tested on the collagen not fixed with fibronectin as a control. These results are shown in Fig. 3. In Fig. 3, the marks ▲, ● and ⊗ represent the results obtained by unmodified atelocollagen fixed with fibronectin, succinylated atelocollagen fixed with fibronectin and methylated atelocollagen fixed with fibronectin, respectively. Further, the marks Δ, 0 and x represent the results obtained by unmodified atelocollagen, succinylated atelocollagen and methylated atelocollagen, respectively, none of which is fixed with fibronectin.

As seen in Fig. 3, the proliferation of L-cells was better with succinylated or methylated atelocollagen substratum than with unmodified atelocollagen in both media, with and without fibronectin. Especially, it is suggested that L-cells proliferated very well on the chemically modified atelocollagen substrata in the medium containing fibronectin.

Example 3

The adhesiveness of macrophages to the substratum was tested by using culture dishes coated with succinylated atelocollagen, methylated atelocollagen and unmodified atelocollagen, respectively. Exudate of mice abdomen was taken and the cells precipitated by centrifuging were dispersed in RPMI 1640 culture medium (made by Nissui Pharmaceutical Co. Ltd.) containing or not containing 10% bovine foetal serum respectively. To each of the culture dishes, 1.5 ml of culture medium containing 1 x $10^6$ cells of macrophage was added and incubated at 37 °C under a 5% $CO_2$ gaseous phase. After incubation, the culture dishes were washed with culture medium, the numbers of not-adhering macrophages were counted and the numbers of adhering macrophages were calculated by subtracting them from the numbers of the inoculated cells.

The relation between the incubation time and adhesion rate of macrophage is shown in Figs. 4 and 5. Fig. 4 shows the result obtained in the presence of bovine foetal serum and Fig. 5 shows the result obtained in its absence. In Fig. 4 and Fig. 5, the marks Δ represents the results obtained from unmodified atelocollagen, the marks 0 represents the results obtained from succinylated atelocollagen and the x represents the results obtained from methylated atelocollagen.

As is clear from Figs. 4 and 5, macrophages adhere to succinylated collagen or methylated collagen in the presence or even in the absence of bovine foetal serum at a high rate, and, when incubated for longer than 60 minutes, adhesion of more than 90% was shown, but macrophages hardly adhered at all to unmodified collagen.

The exudate of the abdomen used in Example 3 contained macrophages in about 50% of all the cells and lymphocytes in the remained 50%. As the lymphocytes did not adhere to the chemically modified collagen, the macrophages could be isolated completely from the lymphocytes by recovering the adhering macrophages.

To succinylated atelocollagen, methylated atelocollagen and unmodified atelocollagen, the macrophages were made to adhere by incubating at 37 °C for 3 hours in the presence of 10% bovine foetal serum. 1.5 ml of phosphate buffer solution containing 0.5 millimole/l of EDTA was added to the culture dishes with adhering macrophages and was kept at 37 °C for 10 minutes to detach macrophages. The detached macrophages were recovered and their numbers counted, and the recovery rate of the macrophages and their purity were calculated.

Table 1

| Substrates | Recovery rate of macrophages (%) | Purity of recovered macrophages (%) |
|---|---|---|
| Succinylated atelocollagen | 41 | 94 |
| Methyalted atelocollagen | 48 | 92 |
| Unmodified atelocollagen | 3 | 88 |

As will be understood from Table 1, macrophages with higher purity were obtained when succinylated or methylated atelocollagen was used as a substratum, and the recovered macrophages were not injured.

## Claims

1. A method for culturing and isolating cells comprising causing animal cells to adhere to succinylated collagen wherein more than 20 mol % of the total amino groups of collagen have been succinylated, or to esterified collagen wherein more than 20 mol % of the total carboxyl groups of collagen have been esterified, in a culture medium, incubating the animal cells and subsequently treating the animal cells with a bivalent cationic chelating agent to isolate and recover the cells.

2. A method according to claim 1, in which macrophages mixed with lymphocytes are incubated, and only the macrophages are selectively isolated and recovered.

3. A method according to claim 1 or 2, in which the bivalent cationic chelating agent is EDTA or EGTA.

4. A method according to any preceding claim in which the adhesion and incubation of animal cells are carried out in the presence of bovine foetal serum or fibronectin.

5. Use of succinylated collagen in which more than 20 mol % of the total amino groups of collagen have been succinylated, or of esterified collagen wherein more than 20 mol % of the carboxyl groups of collagen have been esterified, in a method according to any preceding claim.

## Revendications

1. Procédé pour cultiver et isoler des cellules, qui consiste à faire adhérer des cellules animales à du collagène succinylé dans lequel plus de 20 % molaires des groupes amino totaux du collagène ont été succinylés, ou à du collagène estérifié dans lequel plus de 20 % molaires des groupes carboxyles totaux du collagène ont été estérifiés, dans un milieu de culture, incuber les cellules animales, puis traiter les cellules animales avec un agent chélatant cationique bivalent pour isoler et récupérer les cellules

2. Procédé selon la revendication 1, dans lequel on incube des macrophages mélangés avec des lymphocytes et seuls les macrophages sont sélectivement isolés et récupérés.

3. Procédé selon la revendication 1 ou 2, dans lequel l'agent chélatant cationique bivalent est l'EDTA ou l'EGTA.

4. Procédé selon l'une quelconque des revendications précédentes, dans lequel l'adhésion et l'incubation des cellules animales sont effectuées en présence de sérum foetal de bovin ou de fibronectine.

5. Utilisation de collagène succinylé, dans lequel plus de 20 % molaires des groupes amino totaux du collagène ont été succinylés, ou de collagène estérifié, dans lequel plus de 20 % molaires des groupes carboxyles du collagène ont été estérifiés, dans un procédé selon l'une quelconque des revendications

précédentes.

**Patentansprüche**

1. Verfahren zum Züchten und Isolieren von Zellen, welches darin besteht, tierische Zellen an succinyliertem Kollagen, bei dem mehr als 20 Mol-% der gesamten Aminogruppen des Kollagens succinyliert worden sind, oder an verestertem Kollagen, bei dem mehr als 20 Mol-% der gesamten Carboxylgruppen des Kollagens verestert worden sind, in einem Kulturmedium anhaften zu lassen, die tierischen Zellen zu inkubieren und anschließend die tierischen Zellen mit einem zweiwertigen kationischen Chelatbildner zu behandeln, um die Zellen zu isolieren und zu gewinnen.

2. Verfahren nach Anspruch 1, bei dem mit Lymphozyten vermischte Makrophagen inkubiert werden und lediglich die Makrophagen selektiv isoliert und gewonnen werden.

3. Verfahren nach Anspruch 1 oder 2, worin als zweiwertiger kationischer Chelatbildner EDTA oder EGTA verwendet werden.

4. Verfahren nach irgendeinem der vorhergehenden Ansprüche, worin das Anhaften und Inkubieren der tierischen Zellen in Gegenwart von fetalem Rinderserum oder Fibronectin durchgeführt werden.

5. Verwendung von succinyliertem Kollagen, bei dem mehr als 20 Mol-% der gesamten Aminogruppen des Kollagens succinyliert worden sind, oder von verestertem Kollagen, bei dem mehr als 20 Mol-% der Carboxylgruppen des Kollagens verestert worden sind, bei dem Verfahren gemäß irgendeinem der vorhergehenden Ansprüche.

FIG.1

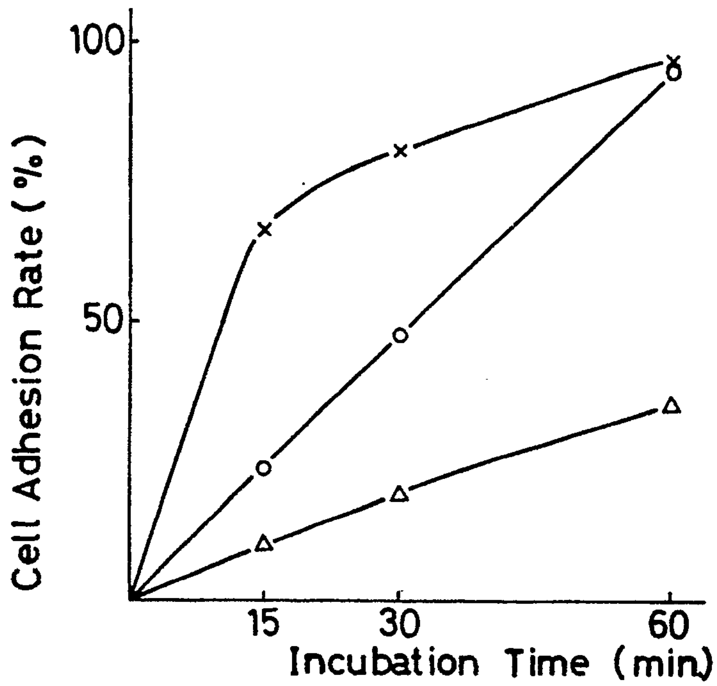
100
50
Cell Adhesion Rate (%)
15
30
60
Incubation Time (min)

FIG.2

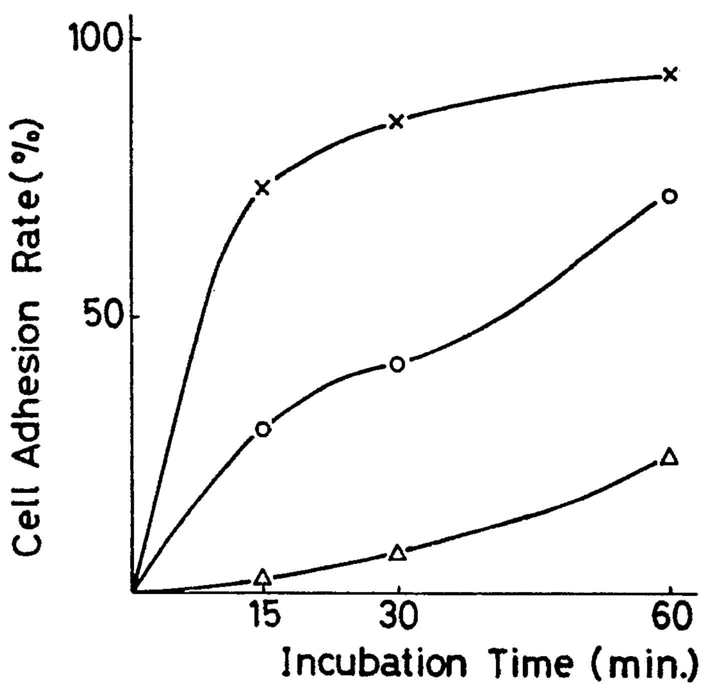
100
50
Cell Adhesion Rate (%)
15
30
60
Incubation Time (min.)

FIG.3

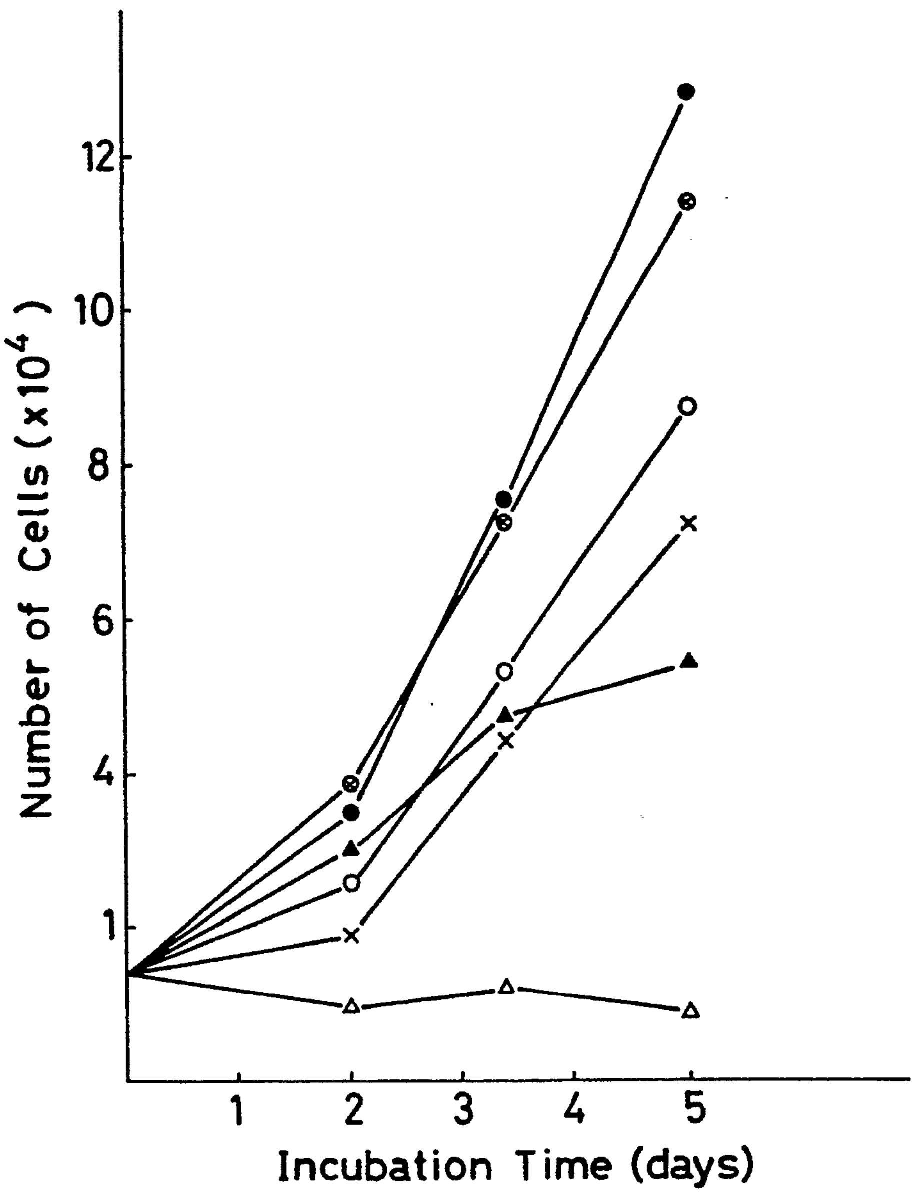

FIG.4

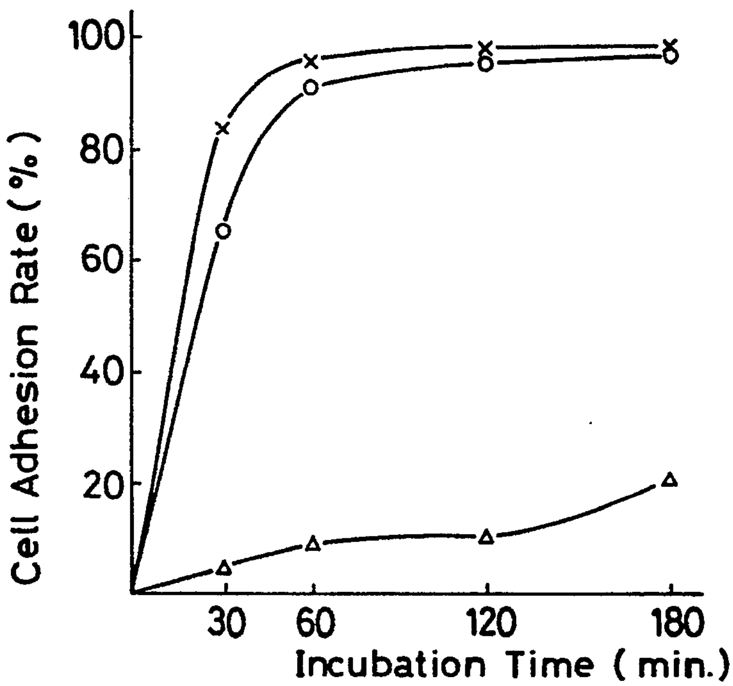
Cell Adhesion Rate (%)
100
80
60
40
20
30
60
120
180
Incubation Time (min.)

FIG.5

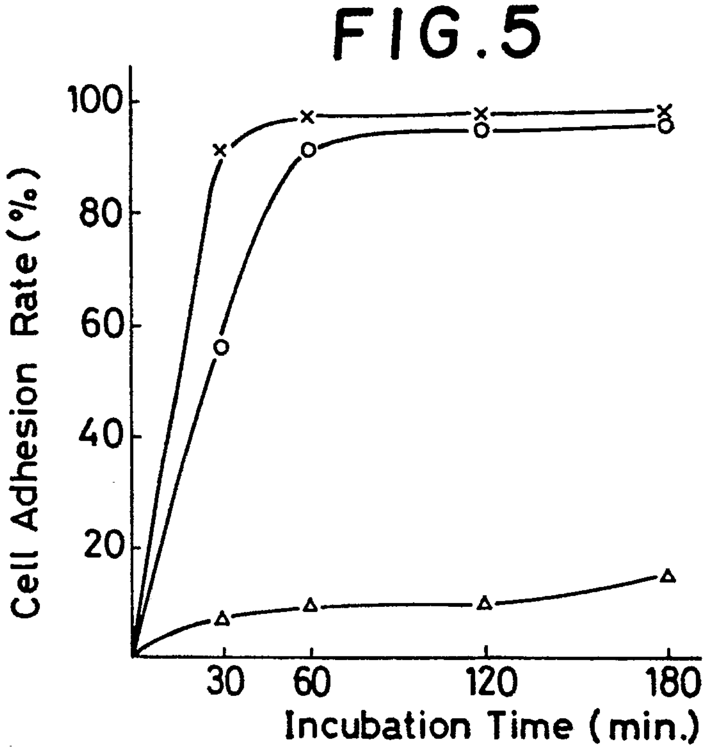
Cell Adhesion Rate (%)
100
80
60
40
20
30
60
120
180
Incubation Time (min.)